(19) Europäisches Patentamt
European Patent Office
Office européen des brevets

(11) EP 1 269 986 B1

(12) # FASCICULE DE BREVET EUROPEEN

(45) Date de publication et mention de la délivrance du brevet:
**25.02.2009 Bulletin 2009/09**

(21) Numéro de dépôt: **02291481.6**

(22) Date de dépôt: **13.06.2002**

(51) Int Cl.:
***A61K 31/506*** *(2006.01)* ***A61K 31/5685*** *(2006.01)*
***A61K 31/616*** *(2006.01)* ***A61P 17/00*** *(2006.01)*
***A61P 17/10*** *(2006.01)* ***A61K 8/37*** *(2006.01)*
***A61K 8/44*** *(2006.01)* ***A61Q 19/00*** *(2006.01)*
***A61Q 19/08*** *(2006.01)* ***A61Q 19/02*** *(2006.01)*

(54) **Compositions comprenant un composé de faible solubilité et un dérivé lipophile d'acide aminé, utilisations et procédés correspondants**

Zusammensetzungen, die eine wenig lösliche Verbindung und ein lipophiles Aminosäurederivat enthalten, und entsprechende Verwendungen und Verfahren

Compositions comprising a compound of low solubility and a lipophilic amino acid derivative, and corresponding uses and processes

(84) Etats contractants désignés:
**DE ES FR GB IT**

(30) Priorité: **26.06.2001 FR 0108428**
**26.06.2001 FR 0108429**
**26.06.2001 FR 0108430**
**26.06.2001 FR 0108431**

(43) Date de publication de la demande:
**02.01.2003 Bulletin 2003/01**

(73) Titulaire: **L'ORÉAL**
**75008 Paris (FR)**

(72) Inventeurs:
- **Guiramand, Carole**
**78350 Jouy en Josas (FR)**
- **Caplain, Dominique**
**75014 Paris (FR)**
- **Chevalier, Véronique**
**94440 Villecresnes (FR)**
- **Simmonet, Jean-Thierry**
**75011 Paris (FR)**
- **Richart, Pascal**
**75013 Paris (FR)**

(74) Mandataire: **Casalonga, Axel**
**Bureau Casalonga & Josse**
**Bayerstrasse 71/73**
**80335 München (DE)**

(56) Documents cités:
**EP-A- 0 336 812** **EP-A- 0 378 936**
**EP-A- 0 662 318** **EP-A- 0 928 608**
**EP-A- 1 002 536** **EP-A- 1 044 676**
**EP-A- 1 092 422** **WO-A-01/26618**
**WO-A-99/10318** **WO-A-99/13857**
**FR-A- 2 802 416** **US-A- 5 736 537**
**US-A- 6 153 208**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).



Printed by Jouve, 75001 PARIS (FR)

## Description

La présente invention se rapporte à des compositions à base de dérivés lipophiles d'acide aminé, à leurs utilisations et à un procédé de solubilisation d'au moins un composé de faible solubilité dans l'eau.

Il est connu d'utiliser des actifs dans des compositions cosmétiques et/ou dermatologiques, par exemple en vue de soigner ou de traiter ou d'apporter des effets bénéfiques à la peau. Cependant, l'utilisation de certains de ces actifs pose un problème dans la mesure où ils se présentent sous forme cristalline et où ils sont difficilement ou pas du tout solubles dans l'eau.

Ainsi, si on les introduit tels quels dans les compositions cosmétiques et/ou dermatologiques, ils restent à l'état de cristaux, ce qui rend l'utilisation de la composition les contenant, inefficace pour le traitement de la peau.

Généralement et pour certains d'entre eux, il est possible de les introduire sous forme hydroalcoolique (eau/éthanol), mais la présence d'alcool n'est pas toujours souhaitable, notamment lors de l'application sur certaines zones du visage, telles que le contour des yeux.

Il subsiste donc le besoin de pouvoir introduire des composés de faible solubilité dans des compositions cosmétiques et/ou dermatologiques, notamment dans la phase aqueuse de telles compositions.

On entend ici par "de faible solubilité", des composés tels que $\delta a > 6\ J^{1/2}\ cm^{-3/2}$ et ayant une solubilité dans l'eau à 25 °C inférieure à 1% poids. On définit généralement une molécule comme étant insoluble dans l'eau à partir d'un certain pourcentage (ici 1 %) quand, au niveau macroscopique, il apparaît un précipité ou que la solution devient trouble, et qu'au niveau microscopique, des cristaux apparaissent.

Tel qu'il est rappelé dans l'ouvrage « Properties of Polymers » de D.W.Van Krevelen 3th edition (Elsevier, 1990) page 200 et sq, la solubilité d'un composé dans un solvant donné est en grande partie déterminée par sa structure chimique.

Le paramètre de solubilité permet de définir une molécule à l'égard des forces d'interaction auxquelles elle participe.

Ce paramètre de solubilité dit "de Hansen" est obtenu par l'équation suivante :

$$\delta^2 = \delta_d{}^2 + \delta_p{}^2 + \delta_h{}^2$$

dans laquelle :

$\delta_d$ représente les forces de dispersion au moment des chocs, dites forces de London ou forces de Van der Waals issues de la formation de dipôles induits lors des chocs moléculaires :

$$\delta_d = \Sigma F_d / V,$$

$\delta_p$ représente les forces de polarisation moléculaires ou forces d'interaction de Debye c'est-à-dire le dipôle permanent généré par la molécule considérée lorsqu'elle est mise en solution, il est calculé par :

$$\delta_p = (\Sigma F_p{}^2)^{1/2} / V,$$

et $\delta_h$ représente les forces d'interaction spécifique comme les liaisons hydrogène, acide/base, donneur/accepteur, calculé par :

$$\delta_h = (\Sigma F_h / V)^{1/2}.$$

Les paramètres $\delta_d$, $\delta_p$ et $\delta_h$ sont exprimés en $(J/cm^3)^{1/2}$.

Chacun des paramètres $\delta_p$ et $\delta_h$ est non nul lorsque la molécule considérée comprend au moins un hétéroatome.

$F_d$, $F_p$, $F_h$ étant les constantes molaires des forces d'interaction des groupes d'atomes constituant les molécules et V le volume molaire qui peut être déterminé par le méthode de Fedors (Polymer Engineering and Science, February, 1974, Vol 14 n°2). V étant la somme des volumes molaires des radicaux qui composent la molécule considérée, la

valeur V pour la plupart des radicaux est donnée dans l'article de Fedors mentionné ci-dessus.

Pour calculer les valeurs de $F_d$, $F_p$, $F_h$ pour une molécule donnée, il suffit de calculer la somme des contributions des radicaux qui composent ladite molécule. Les valeurs de $F_d$, $F_p$, $F_h$ ont été établies pour la plupart des radicaux, l'ouvrage « Properties of Polymers » de D.W.Van Krevelen cité ci-dessus contient notamment des tableaux présentant ces valeurs pour de nombreux radicaux.

Dans le but d'obtenir une expression de la solubilité en deux composantes, on a également défini:

$$\delta_a^2 = \delta_p^2 + \delta_h^2 = \delta^2 - \delta_d^2$$

d'où

$$\delta_a = (\delta_p^2 + \delta_h^2)^{1/2}$$

Ces notions de solubilité et de cohésion sont définies en particulier dans l'ouvrage : « Properties of Polymers » de D.W.Van Krevelen 3th edition (Elsevier, 1990) chapitre 7 et dans l'article "A method for estimating both the solubility parameters and molar volumes of liquids" R.F. Fedors, Polymer Engineering and Science, February 1974 vol.14 n°2 p147-154.

Au sens de la présente invention, les inventeurs ont défini les molécules de faible solubilité dans l'eau comme étant des molécules ayant une solubilité dans l'eau à 25°C inférieure à 1% poids et telles que $\delta a > 6\ J^{1/2}\ cm^{-3/2}$.

Il est souvent nécessaire de pouvoir solubiliser aisément dans un milieu physiologiquement acceptable des molécules de faible solubilité dans l'eau. Les solutions et compositions ainsi obtenues sont généralement faciles à mettre en oeuvre et elles peuvent être appliquées avec un minimum d'inconfort. En outre, il est souvent nécessaire de pouvoir solubiliser une quantité suffisante de ces molécules de faible solubilité en vue de leur utilisation cosmétique ou dermatologique, sans recristallisation de ces molécules ni perte de solubilité de la composition les contenant. Cette instabilité résulterait en effet en une perte d'efficacité plus ou moins importante de ces compositions et/ou en une modification de leur aspect, qui risquerait de détourner l'utilisateur de celles-ci

La Demanderesse a maintenant découvert que des dérivés lipophiles d'acide aminé permettaient d'augmenter de façon inattendue la solubilisation de ces molécules de faible solubilité et de garder ces molécules solubilisées dans des compositions qui sont de ce fait stables.

La présente invention a donc pour objet un procédé de solubilisation d'au moins un composé de faible solubilité de formule (I'), ledit procédé comprenant l'étape essentielle de mélanger le composé de faible solubilité avec au moins un dérivé lipophile d'acide aminé, ainsi qu'une composition comprenant, dans un milieu physiologiquement acceptable, au moins un composé ayant une solubilité dans l'eau à 25°C inférieure à 1% et tel que $\delta a > 6\ J^{1/2}\ cm^{-3/2}$ de formule (I') et au moins un dérivé lipophile d'acide aminé.

Par "milieu physiologiquement acceptable" au sens de la présente demande, on entend un milieu compatible avec la peau y compris le cuir chevelu, les muqueuses, les yeux et/ou les cheveux.

A titre d'illustration, les dérivés lipophiles d'acide aminé utilisés au sens de la présente demande ont permis de solubiliser :

- plus de 20% de dérivés aminophénol sans recristallisation après trois semaines à 25° C,
- plus de 15% de dérivés d'acide salicylique sans recristallisation après trois semaines à 25° C,
- environ 4% de diosgénine ou hécogénine acétate sans recristallisation après 24 heures à 25°C.

Dans le cas d'une mise en émulsion, l'utilisation d'un de ces dérivés lipophiles d'acide aminé permet de s'affranchir de la limite traditionnellement imposée par le taux de solubilisant, la cosméticité de ces solubilisants étant généralement rédhibitoire. Dans le cadre de la présente invention, les émulsions gardent une cosméticité très acceptable malgré de forts taux de solubilisant.

Le dérivé lipophile d'acide aminé utilisé selon l'invention est un ester d'acide aminé de formule :

$$R'_1\ (CO)N(R'_2)CH(R_3)(CH_2)n(CO)OR'_4$$

dans laquelle :

n est en entier égal à 0,1 ou 2,

$R'_1$ représente un radical alkyle ou alcényle en $C_5$ à $C_{21}$, linéaire ou ramifié, ,
$R'_2$ représente un atome d'hydrogène ou un groupe alkyle en $C_1$ à $C_3$,
$R'_3$ représente un radical choisi dans le groupe formé par un atome d'hydrogène, un groupe méthyle, un groupe éthyle, un radical alkyle linéaire ou ramifié en $C_3$ ou $C_4$,
$R'_4$ représente un radical alkyle en $C_1$ à $C_{10}$ ou alcényle en $C_2$ à $C_{10}$, linéaire ou ramifié, ou un reste stérol.

De préférence, le groupement $R'_1(CO)$- est un groupement acyle d'un acide choisi dans le groupe formé par l'acide caprique, l'acide laurique, l'acide myristique, l'acide palmitique, l'acide stéarique, l'acide béhénique, l'acide linoléique, l'acide linolénique, l'acide oléique, l'acide isostéarique, l'acide 2-éthylhexanoïque, les acides gras d'huile de coco, les acides gras d'huile de palmiste. Ces acides gras peuvent en outre présenter un groupe hydroxyle. De manière encore plus préférée, il s'agira de l'acide laurique.

La partie $-N(R'_2)CH(R'_3)(CH_2)_n(CO)-$ de l'ester d'aminoacide est de préférence choisie parmi les aminoacides suivants : glycine, alanine, valine, leucine, isoleucine, sérine, thréonine, proline, hydroxyproline, β-alanine, acide aminobutyrique, acide aminocaproique, sarcosine, ou N-méthyl-β-alanine.

De manière encore plus préférée, il s'agira de la sarcosine.

La partie des esters aminoacides correspondant au groupe $OR'_4$ peut être obtenue à partir des alcools choisis dans le groupe formé par le méthanol, éthanol, propanol, isopropanol, butanol, tert-butanol, isobutanol, 3-méthyl-1-butanol, 2-méthyl-1-butanol, huile de fusel, pentanol, héxanol, cyclohéxanol, octanol, 2-éthylhéxanol, décanol, alcool laurylique, alcool myristique, alcool cétylique, alcool cétostéarylique, alcool stéarylique, alcool oléique, alcool béhénylique, alcool de jojoba, alcool 2-héxadécylique, alcool 2-octyldodécanol et alcool isostéarylique.

Ces esters d'acide aminé peuvent en particulier être obtenus à partir de sources naturelles en acides aminés. Dans ce cas, les acides aminés proviennent d'hydrolyse de protéines naturelles végétales (avoine, blé, soja, palme, coco) et conduisent alors nécessairement à des mélanges d'acides aminés qui devront ensuite être estérifiés puis N-acylés. La préparation de tels acides aminés est plus particulièrement décrite dans la demande de brevet FR 2 796 550.

L'ester d'acide aminé plus particulièrement préféré pour son utilisation dans la présente invention est le N-lauroylsarcosinate d'isopropyl de formule :

$$CH_3\text{-}(CH_2)_{10}CO\text{-}N(CH_3)\text{-}CH_2\text{-}COO\text{-}\ CH_2\text{-}(\ CH_3)_2.$$

Les esters d'aminoacide utilisés de préférence au sens de la présente invention, ainsi que leur synthèse sont décrits dans les demandes de brevets EP 1 044 676 et EP 0 928 608 de la société AJINOMOTO CO.

De manière générale, le ou les dérivés lipophiles d'acides aminés représentent de 0,01 à 90% en poids, de préférence de 0,1 à 50% en poids et de manière encore plus préférée de 0,1 à 30% en poids par rapport au poids total de la composition.

Les composés de faible solubilité dans l'eau au sens de la présente invention sont des dérivés d'acide salicylique.

De manière générale, le composé de faible solubilité représente de 0,001 à 30% en poids, et préférentiellement de 0,05 à 15% en poids par rapport au poids total de la composition.

Au sens de la présente demande, les dérivés d'acide salicylique de faible solubilité sont les dérivés de formule I' ou les sels monovalents, divalents ou les mélanges de ces dérivés :

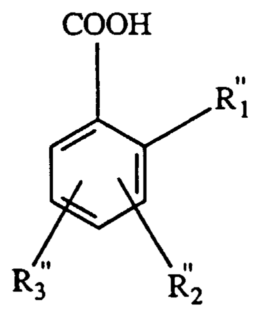

(I')

dans laquelle

$R''_1$ représente un radical hydroxyle ou un ester de formule

$$-O-CO-R''_4$$

dans laquelle $R''_4$ est un radical aliphatique, saturé ou insaturé comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone,
$R''_2$ et $R''_3$ indépendamment l'un de l'autre se trouvent en l'une des positions 3, 4, 5 ou 6 sur le noyau benzénique et représentent indépendamment l'un de l'autre un atome d'hydrogène ou un radical:

$$-(O)_n-(CO)_m-R''_5$$

dans lequel n et m, indépendamment l'un de l'autre, sont chacun un entier égal à 0 ou 1, à la condition que $R''_2$ et $R''_3$ ne soient pas simultanément des atomes d'hydrogène,
et $R''_5$ représente un hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone, un radical aromatique comprenant de 6 à 10 atomes de carbone.

De manière préférée, le dérivé d'acide salicylique est tel que $R''_5$ représente un radical aliphatique saturé comprenant de 3 à 15 atomes de carbone.

De préférence, le dérivé d'acide salicylique est tel que $R''_1$ représente un radical hydroxyle.

De préférence, le dérivé d'acide salicylique est tel que $R''_3$ est en position 5 du noyau benzénique et $R''_2$ représente un atome d'hydrogène.

Selon un mode de réalisation préféré de l'invention, le dérivé d'acide salicylique est choisi parmi les dérivés n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-5-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-oléoyl-5-salicylique, benzoyl-5-salicylique, leurs sels monovalents et divalents et leurs mélanges.

Selon un autre mode de réalisation préféré, les compositions comprennent un sel de dérivé d'acide salicylique de formule I' choisi dans parmi les sels de strontium, de calcium, de magnésium, de baryum et de manganèse. De manière encore plus préférée, ce sel de dérivé d'acide salicylique est choisi parmi le sel de strontium de l'acide 5-octanoyl salicylique, le sel de calcium de l'acide 5-octanoyl salicylique, le sel de magnésium de l'acide 5-octanoyl salicylique et leurs mélanges.

Ces dérivés sont connus dans l'art antérieur, en particulier la demande de brevet EP 662 318 concerne l'utilisation de tels dérivés d'acide salicylique pour fabriquer des compositions cosmétiques et/ou dermatologiques pour le traitement du corps et du visage notamment pour le traitement de l'acné et du vieillissement de la peau. Les demandes de brevets EP 0 662 318, EP 987 011 décrivent des procédés de préparation de ces tels dérivés d'acide salicylique.

Les dérivés d'acide salicylique sont d'un grand intérêt notamment pour prévenir ou réparer les principales manifestations du vieillissement cutané que sont les ridules et rides, la désorganisation du « grain » de la peau, la modification du teint de la peau et la perte de fermeté et de tonicité de la peau. Cependant, l'utilisation de ces dérivés pose un problème dans la mesure où, lorsqu'ils sont introduits tels quels dans les compositions topiques, ils ne se solubilisent pas et restent à l'état de cristaux, ce qui rend l'utilisation de la composition les contenant, inefficace pour le traitement de la peau.

Généralement, ces dérivés sont mis en solution dans les alcools inférieurs comme l'éthanol ou l'isopropanol ou des solvants tels que l'octyldodécanol, certains glycols, les alcools gras à chaîne courte (inférieurs à C12). Cependant, ces alcools inférieurs présentent l'inconvénient de dessécher et d'irriter la peau ; on préfère donc éviter de les utiliser dans les produits de soin du corps et/ou du visage. En outre, ces solubilisants ne peuvent être introduits qu'en de petites quantités sous peine d'altérer les qualités cosmétiques (dessèchement de la peau) et la stabilité des compositions les contenant.

La concentration en dérivés d'acide salicylique de la composition selon la présente invention est comprise entre 0,001 et 15% en poids, de préférence entre 0,1 et 5% en poids par rapport au poids total de la composition. La quantité d'esters d'acide aminé dépendra de la quantité de dérivés d'acide salicylique à solubiliser. Elle pourra être comprise entre 0,01 et 90% en poids, et de préférence entre 0,1 et 50% en poids par rapport au poids total de la composition.

La composition selon l'invention comprenant au moins un dérivé salicylique peut être utilisée comme composition cosmétique ou dermatologique, et notamment pour le soin, la protection, le nettoyage et/ou le maquillage des matières kératiniques des êtres humains (peau, lèvres, fibres kératiniques telles que cheveux et cils), et notamment pour lutter contre les signes du vieillissement cutané et/ou pour lisser la peau du visage et/ou du corps et/ou pour traiter les rides et les ridules de la peau et/ou pour stimuler le processus de renouvellement épidermique et/ou pour dépigmenter et blanchir la peau et/ou pour traiter l'acné et/ou pour traiter les désordres cutanés.

Par désordres cutanés, on entend en particulier le zona, les brûlures, l'eczéma, la démodécie, l'ulcère cutané, la fibrose, le contrôle des cicatrisations, le psoriasis, les prurits, les dermatites, l'ichtyose, les cors et les verrues.

Aussi, l'invention a encore pour objet l'utilisation cosmétique de la composition cosmétique telle que définie ci-dessus pour la protection, le soin, le nettoyage et/ou le maquillage de la peau et/ou des muqueuses et/ou des fibres kératiniques.

L'invention a encore pour objet un procédé de traitement cosmétique pour la protection, le soin, le nettoyage et/ou le maquillage de la peau et/ou des muqueuses et/ou des fibres kératiniques, consistant à appliquer sur la peau et/ou les muqueuses et/ou les fibres kératiniques, la composition selon l'invention.

L'invention a encore pour objet un procédé de traitement cosmétique pour lutter contre les signes du vieillissement cutané et/ou améliorer l'éclat du teint et/ou lisser la peau du visage et/ou du corps et/ou traiter les rides et les ridules de la peau et/ou stimuler le processus de renouvellement épidermique et/ou pour dépigmenter et/ou blanchir la peau, consistant à appliquer sur la peau la composition contenant un dérivé d'acide salicylique selon l'invention.

L'invention a aussi pour objet l'utilisation de la composition selon l'invention pour la fabrication d'une composition thérapeutique dermatologique destinée à lutter contre les signes du vieillissement cutané et/ou lutter contre l'acné et/ou lutter contre les désordres cutanés.

Le rapport en poids dérivé d'acide salicylique/ester d'aminoacide est de préférence de 0,001 /99,999 à 35/65 et de préférence de 0,1/99,9 à 30/70.

La composition cosmétique selon la présente invention peut se présenter sous les formes normalement utilisées en cosmétique.

Elle peut se présenter sous toutes les formes normalement utilisées pour une application topique, notamment sous forme d'une solution hydroalcoolique, d'une émulsion huile-dans-eau ou eau-dans-huile ou multiple, d'un gel huileux, ou d'un produit anhydre liquide, pâteux ou solide ou sous forme de dispersion en présence de sphérules, ces sphérules peuvent être des nanoparticules polymériques telles que des nanosphères ou des nanocapsules ou des vésicules lipidiques de type ionique ou non-ionique. Ces compositions sont préparées selon les méthodes usuelles.

Cette composition peut être plus ou moins fluide et avoir l'aspect d'une crème blanche ou colorée, d'une pommade, d'un lait, d'une lotion, d'un sérum, d'une pâte, d'une mousse. Elle peut éventuellement être appliquée sur la peau ou les cheveux sous forme d'aérosol. Elle peut également se présenter sous forme solide, et par exemple sous forme de stick.

De façon connue, les compositions cosmétiques et dermatologiques selon l'invention peuvent contenir également les adjuvants habituels dans les domaines cosmétique et dermatologique, tels que des gélifiants hydrophiles ou lipophiles, des actifs hydrophiles ou lipophiles, des conservateurs, des antioxydants, des solvants, des parfums, des fibres, des charges, des filtres, des pigments, des absorbeurs d'odeur et des matières colorantes. Les quantités de ces différents adjuvants sont celles classiquement utilisées dans les domaines considérés, et par exemple de 0,01 à 50 % du poids total de la composition. Ces adjuvants, selon leur nature, peuvent être introduits dans la phase grasse, dans la phase aqueuse, dans des vésicules lipidiques et/ou dans des nanoparticules.

Bien entendu, l'homme du métier veillera à choisir ces éventuels composés complémentaires, actifs ou non actifs, et/ou leur quantité, de telle manière que les propriétés avantageuses des dérivés de faible solubilité ne soient pas, ou sensiblement pas, altérées par l'adjonction envisagée.

Lorsque la composition selon l'invention est une émulsion, la proportion de la phase grasse peut aller de 0,5 à 80 % en poids, et de préférence de 5 à 50 % en poids par rapport au poids total de la composition. Les huiles, les émulsionnants et les coémulsionnants utilisés dans la composition sous forme d'émulsion sont choisis parmi ceux classiquement utilisés dans le domaine considéré.

La phase grasse ou phase huileuse contient habituellement au moins une huile. Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arana, de tournesol, de ricin, d'avocat, les triglycérides des acides caprylique/caprique comme ceux vendus par la société Stearineries Dubois ou ceux vendus sous les dénominations Miglyol 810, 812 et 818 par la société Dynamit Nobel, l'huile de jojoba, l'huile de beurre de karité ;
- les esters et les éthers de synthèse, notamment d'acides gras, comme les huiles de formules $R^6COOR^7$ et $R^6OR^7$

dans laquelle $R^6$ représente le reste d'un acide gras comportant de 8 à 29 atomes de carbone, et $R^7$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononaoate de diéthylèneglycol ; et les esters de pendaérythritol comme le tétraisostéarate de pentaérythrityle ;

- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que l'huile de parléam ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoxylés et notamment éthoxylés tels que l'oleth-12 ;
- les huiles fluorées partiellement hydrocarbonées et/ou siliconées comme celles décrites dans le document JP-A-2 295912. Comme huiles fluorées, on peut citer aussi le perfluorométhylcyclopentane et le perfluoro-1,3-diméthylcyclohexane, vendus sous les dénominations de « FLUTEC PC1® » et « FLUTEC PC3® » par la Société BNFL Fluorochemicals ; le perfluoro-1,2-diméthylcyclobutane ; les perfluoralcanes tels que le dodécafluoropentane et le tétradécafluorohexane, vendus sous les dénominations de « PF 5050® » et « PF 5060® » par la Société 3M, ou encore le bromoperfluorooctyle vendu sous la dénomination « FORALKYL® » par la Société Atochem ; le nanofluorométhoxybutane vendu sous la dénomination « MSX 4518® » par la Société 3M et le nanofluoroéthoxyisobutane ; les dérivés de perfluoromorpholine, tels que la 4-trifluorométhyl perfluoromorpholine vendue sous la dénomination « PF 5052® » par la Société 3M ;
- les huiles de silicone comme les polyméthylsiloxanes (PDMS) volatiles ou non à chaîne siliconée linéaire ou cyclique, liquides ou pâteaux à température ambiante, notamment les cyclopolydiméthylsiloxanes (cyclométhicones) telles que la cyclohexasiloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, les polyméthylphénylsiloxanes ; et leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

Les autres corps gras pouvant être présents dans la phase huileuse sont par exemple les acides gras comportant de 8 à 30 atomes de carbone, comme l'acide stéarique, l'acide laurique, l'acide palmitique et l'acide oléique ; les cires comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylènes, les cires de Fischer-Tropsch ; les gommes telles que les gommes de silicone (diméthiconol) ; les résines de silicone telles que la triflurorométhyl-C1-4-alkyldimethicone et la trifluoropropyldimethicone ; et les élastomères de silicone comme les produits commercialisés sous les dénominations « KSG » par la société Shin-Etsu, sous les dénominations « Trefil », « BY29 » ou « EPSX » par la société Dow Corning ou sous les dénominations « Gransil » par la société Grant Industries.

Ces corps gras peuvent être choisis de manière variée par l'homme du métier afin de préparer une composition ayant les propriétés, par exemple de consistance ou de texture, souhaitées. Les émulsions peuvent contenir au moins un émulsionnant choisi parmi les émulsionnants amphotères, anioniques, cationiques ou non ioniques, utilisés seuls ou en mélange.

Quant la composition est une émulsion elle contient en général au moins un émulsionnant. Les émulsionnants sont choisis de manière appropriée suivant l'émulsion à obtenir eau dans l'huile (E/H) ou huile dans l'eau (H/E).

Pour les émulsions H/E, on peut utiliser par exemple comme émulsionnant, un émulsionnant non ionique, comme les esters et éthers d'oses tels que le stéarate de sucrose, le cocoate de sucrose, et le mélange de stéarate de sorbitan et de cocoate de sucrose commercialisé par la société ICI sous la dénomination d'Arlatone 2121® ; les esters de polyol, notamment de glycérol ou de sorbitol, tels que le stéarate de glycéryle, le stéarate de polyglycéryl-2, le stéarate de sorbitan ; les éthers de glycérol ; les éthers oxyéthylénés et/ou oxypropylénés tels que l'éther oxyéthyléné, oxypropyléné de l'alcool laurique à 25 groupes oxyéthylénés et 25 groupes oxypropylénés (nom CTFA « PPG-25 laureth-25 ») et l'éther oxyéthyléné du mélange d'alcools gras en C12-C15 comportant 7 groupes oxyéthylénés (nom CTFA « C12-C15 Pareth-7 ») ; les polymères d'éthylène glycol, tels que le PEG-100, et leurs mélanges.

Pour les émulsions E/H, on peut citer par exemple comme émulsionnant, les esters gras de polyol, notamment de glycérol ou de sorbitol, et notamment les esters isostéariques, oléiques et ricinoléiques de polyol, tels que le mélange

de petrolatum, d'oléate de polyglycéryl-3,d'isostéarate de glycéryle ; l'huile de ricin hydrogénée et d'ozokérite, vendu sous la dénomination PROTEGIN W® par la société Goldschmidt, l'isostéarate de sorbitan, le di-isostéarate de polyglycéryle, le sesqui-isostéarate de polyglycéryle-2 ; les esters et éthers d'oses tels que le « Methyl glucose dioleate » ; les esters gras tels que le lanolate de magnésium ; les dimethicone copolyols et alkyl-dimethicone copolyols tels que le Laurylmethicone copolyol vendu sous la dénomination DOW CORNING 5200 FORMULATION AID par la société Dow Corning et le Cetyl diméthicone copolyol vendu sous la dénomination ABIL EM 90® par la société Goldschmidt, et leurs mélanges.

Les émulsionnants peuvent être introduits tels quels ou sous forme de mélange avec d'autres émulsionnants et/ou avec d'autres composés tels que des alcools gras ou des huiles.

Comme actifs utilisables dans la composition de l'invention, on peut citer par exemple les agents hydratants et par exemple les hydrolysats de protéines et les polyols tels que la glycérine, les glycols comme les polyéthylène glycols, et les dérivés de sucre ; les extraits naturels ; les oligomères procyannidoliques ; les vitamines comme la vitamine A (rétinol), la vitamine C (acide ascorbique), la vitamine E (tocophérol), la vitamine B5 (panthénol), la vitamine B3 (niacinamide) ; la vitamine K ; l'urée ; la caféine ; les dépigmentants tels que l'acide kojique et l'acide caféique ; l'acide salicylique (pour les compositions ne contenant pas déjà un dérivé d'acide salicylique); les alpha-hydroxyacides tels que l'acide lactique et l'acide glycolique ; les rétinoïdes tels que les caroténoïdes ; les filtres solaires; les charges, pigments, colorants, agents kératolytiques, conservateurs, anti-oxydants, parfums, l'hydrocortisone ; la mélatonine ; les extraits d'algues, de champignons, de végétaux, de levures, de bactéries ; les protéines hydrolysées, partiellement hydrolysées ou non hydrolysées, les enzymes ; les actifs anti-bactériens pour le traitement des peaux grasses comme le 2,4,4'-trichloro-2'-hydroxy diphényl éther (ou triclosan), le 3,4,4'-trichlorocarbanilide (ou triclocarban), acide azélaique, peroxyde de benzoyle ; les agents matifiants comme les fibres ; les agents tenseurs ; les azurants optiques ; et leurs mélanges, comme par exemple, le mélange de vitamines (A et C ou A + C + E par exemple).

Le ou les actifs peuvent être présents en une concentration allant de 0,01 à 20%, de préférence de 0,1 à 10% et mieux de 0,5 à 5% du poids total de la composition.

EXEMPLES

Dans le tableau suivant sont présentées des molécules de faible solubilité dans l'eau au sens de la présente invention, elles sont peu solubles dans l'eau notamment à température ambiante et présentent un $\delta a > 6 J^{1/2} cm^{-3/2}$.

| | Solubilité dans l'eau à 25°C | $\delta a$ en $J^{1/2}.cm^{-3/2}$ |
|---|---|---|
| N-Ethoxycarbonyl para aminophénol * | < 1% | 16.1 |
| N-cholesteryloxycarbonyl-para aminophénol * | | 8.7 |
| (3beta, 5alpha, 25r)-3-hydroxyspirostan-12-one (Hécogénine acétate) * | | 6.7 |
| 5-spirosten-3-beta-ol (Diosgénine) | | 8.6 |
| acide n-octanoyl-5 salicylique | | 11.3 |
| DHEA * | | 9.9 |
| 2-amino 4-dodecylamino pyrimidine 3-oxyde * | | 8.9 |
| * Examples hors invention | | |

Les exemples qui suivent illustrent l'invention sans en limiter la portée.

**1 - Solubilité simple**

Protocole : les dérivés de faible solubilité dans l'eau sont pesés et placés dans un pilulier hermétique. La quantité de solvant requise est ajoutée. La suspension est agitée (agitation magnétique) jusqu'à 80° C pendant 1 h au plus.

La dissolution ou la non dissolution de l'actif, ainsi que l'évolution dans le temps sont reportées dans le tableau ci-après.

La non solubilité de l'actif dans le solvant se caractérise macroscopiquement par un précipité ou juste une solution trouble, et microscopiquement par la présence de cristaux.

Le solvant utilisé est le N-lauroyl-sarcosinate d'isopropyle.

| Définition | Taux | Solubilisation à T0 | Stabilité dans le temps à 25°C |
|---|---|---|---|
| N-cholesteryloxy-carbonyl- para aminophénol | 5% | | Limpide pendant 7 jours |
| | 9% | | Limpide pendant 4 jours |
| N-Ethoxycarbonyl-para aminophénol | 5% | Aucune recristallisation | Limpide pendant 18 jours |
| | 20% | | Limpide pendant 6 jours |
| | 23% | | Limpide pendant 6 jours |
| | 26% | | Limpide pendant 1 jour |
| DHEA | 20% | Aucune recristallisation | - |
| 2-amino 4-dodécylamino pyrimidine 3-oxyde | 1% | Aucune recristallisation | Limpide pendant 1 jour |
| DIOSGENINE | 2% | Aucune recristallisation | Limpide pendant 3 semaines |
| HECOGENINE ACETATE | 2% | Aucune recristallisation | Limpide pendant 1 jour |
| Acide-octanoyl-5-salicylique | 5% | Aucune recristallisation | Limpide pendant 7 jours |
| | 9% | | Aucune Limpide pendant 4 jours |
| | 27% | | Limpide |

**2 - Mise en émulsion**

La solubilisation des actifs par les solvants (solubilisants) cités ci-dessus, a été vérifiée en émulsion et ainsi permet de formuler des compositions stables.

Les émulsions réalisées sont des H/E, avec comme tensioactif du Simulsol 165 (mélange de Glyceryl Searate et PEG-100 Stearate).

La stabilité physio-chimique des émulsions est vérifiée par un contrôle macroscopique, microscopique, du pH et de la viscosité, au bout de 24 heures et au cours du temps. La composition selon la présente invention peut se présenter sous les formes normalement utilisées en cosmétique.

**Exemples de compositions**

| | | Acide n-octa-noyl-5 salicylique | |
|---|---|---|---|
| PHASE | Nom CTFA | Exemple conforme | Ex. comparatif |
| A | Conservateur | 0,2 | 0,2 |
| | Disodium EDTA | 0,15 | 0,15 |
| | Glycérine | 3 | 3 |
| | Eau | qsp100 | qsp100 |
| B | Glycéryle stéarate et PEG-100 stéarate | 0,3 | 0,3 |
| | Alcool cétylique | 0,4 | 0,4 |
| | Conservateur | 0,1 | 0,1 |
| | Triclosan | 0,1 | 0,1 |
| C | N-cholestérylcarbony 1 paraaminophénol | - | - |
| | N-ethoxycarbonyl-paraamino-phénol | - | - |
| | Acide n-octanoyle-5 salicylique | 1 | 1 |
| | Lauroylsarcosinate d’isopropyle | 10 | - |

(suite)

| | | Acide n-octa-noyl-5 salicylique | |
|---|---|---|---|
| PHASE | Nom CTFA | Exemple conforme | Ex. comparatif |
| D | Polymère réticulé (acrylates / $C_{10}$-$C_{30}$ alkyl acrylate) | 0,5 | 0,5 |
| | Cyclohexasiloxane | 5 | 5 |
| E | Eau | 10 | 10 |
| | Alcool | 5 | 5 |
| F | Polyacrylamide et isoparaffine en $C_{13}$-$C_{14}$ et laureth-7 | 1 | 1 |
| G | triéthanolamine | 1,03 | 1,03 |
| | Eau | 7 | 7 |

**MODE OPERATOIRE:**

**Phase A**

On chauffe à 85° C. On agite pour solubiliser les conservateurs. Puis on redescend la température à 75°C pour faire l'émulsion.

**Phase B**

On chauffe à 75°C et on homogénise jusqu'à dissolution complète.
Réalisation de l'émulsion : On verse B dans A à 75°C sous agitation pendant 15 mn.

**Phase C**

On homogénise jusqu'à dissolution complète à 30°C et on ajoute dans l'émulsion (A+B) à 60°C.

**Phase D**

On disperse à température ambiante, et on ajoute dans l'émulsion (A+B+C) à 60°C. On homogénise .

**Phases E, F et G**

On les prépare à température ambiante, puis on les ajoute à l'émulsion (A+B+C+D) à 40°C, et on homogénise sous agitation.
On laisse refroidir l'émulsion à 25°C.

Il a été montré que la solubilisation des dérivés de faible solubilité par des esters d'aminoacide est bien réalisable en émulsion afin de confirmer qu'il est possible de formuler les compositions cosmétiques stables contenant de tels dérivés.

Les émulsions réalisées ci-dessus sont des huiles dans l'eau avec du Simulsol 165 commercialisé par la société SEPPIC en tant que tensioactif, le Simulsol 165 étant un mélange de glycéryl stéarate et de PEG-100 stéarate.

La stabilité physico-chimique des émulsions est vérifiée par un contrôle macroscopique, microscopique, du pH et de la viscosité au bout de 24 heures.

| | Avec 10% de N-lauroylsarcosinate d'isopropyl (conformes) | Sans N-lauroylsarcosinate d'isopropyl |
|---|---|---|
| 1% d'acide N-octanoyl-5 salicylique | Exemple<br>Emulsion sans cristaux, elle reste conforme jusqu'à 2 mois à 4/25 et 45°C | Exemple comparatif<br>Emulsion fine avec cristaux dès T 24 heures |

Les compositions conformes à l'invention ne contiennent pas de cristaux et sont stables dans le temps et en

température, alors que les compositions des exemples comparatifs présentent des cristaux dès 24 heures après leur préparation.

## Revendications

**1.** Composition, cosmétique ou dermatologique, comprenant, dans un milieu physiologiquement acceptable, au moins un composé de faible solubilité dans l'eau, c'est-à-dire de solubilité dans l'eau à 25°C inférieure à 1% poids et tel que $\delta a > 6\ J^{1/2}\ cm^{-3/2}$, ledit composé de faible solubilité dans l'eau étant choisi parmi les dérivés d'acide salicylique de formule I' ou un sel monovalent, divalent ou les mélanges de dérivés de formule I' :

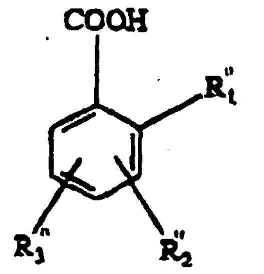

(I')

dans laquelle

$R''_1$ représente un radical hydroxyle ou un ester de formule

$$-O-CO-R''_4$$

dans laquelle $R''_4$ est un radical aliphatique, saturé ou insaturé comprenant de 1 à 26 atomes de carbone, et de préférence de 1 à 18 atomes de carbone, une fonction amine ou thiol éventuellement substituée par un radical alkyle comprenant de 1 à 18 atomes de carbone, et de préférence de 1 à 12 atomes de carbone.
$R''_2$ et $R''_3$ indépendamment l'un de l'autre se trouvent en l'une des positions 3, 4, 5 ou 6 sur le noyau benzénique et représentent indépendamment l'un de l'autre an atome d'hydrogène ou un radical:

$$-(O)_n-(CO)_m-R''_5$$

dans lequel n et m, indépendamment l'un de l'autre, sont chacun un entier égal à 0 ou I, à la condition que $R''_2$ et $R''_3$ ne soient pas simultanément des atomes d'hydrogène,
et $R''_5$ représente un hydrogène, un radical aliphatique saturé comprenant de 1 à 18 atomes de carbone, linéaire, ramifié ou cyclisé, un radical insaturé comprenant de 3 à 18 atomes de carbone, portant une à neuf doubles liaisons conjuguées ou non, les radicaux pouvant être substitués par au moins un substituant choisi parmi les atomes d'halogène (fluor, chlore, brome, iode), les radicaux trifluorométhyle, hydroxyle sous forme libre ou estérifiée par un acide comprenant de 1 à 6 atomes de carbone, ou carboxyle libre ou estérifié par un alcool inférieur comprenant de 1 à 6 atomes de carbone, un radical aromatique comprenant de 6 à 10 atomes de carbone

et au moins un dérivé lipophile d'acide aminé, qui est un ester d'acide aminé de formule ;

$$R'_1\ (CO)N(R'_2)CH(R'_3)(CH_2)n(CO)OR'_4$$

dans laquelle :

n est en entier égal à 0,1 ou 2,
$R'_1$ représente un radical alkyle ou alcényle en $C_5$ à $C_{21}$, linéaire ou ramifié,
$R'_2$ représente un atome d'hydrogène ou un groupe alkyle en $C_1$ à $C_3$,

R'$_3$ représente un radical choisi dans le groupe formé par un atome d'hydrogène, un groupe méthyle, un groupe éthyle, un radical alkyle linéaire ou ramifié en $C_3$ ou $C_4$,
R'$_4$ représente un radical alkyle en $C_1$ à $C_{10}$ ou alcényle en $C_2$-$C_{10}$, linéaire ou ramifié, ou un reste stérol.

2. Composition selon la revendication 1 telle que ledit ester d'acide aminé est le N-lauroylsarconisate d'isopropyle

$$CH_3\text{-}(CH_2)_{10}CO\text{-}N(CH_3)\text{-}CH_2\text{-}COO\text{-}CH\text{-}(CH_3)_2.$$

3. Composition selon l'une quelconque des revendications 1 ou 2 **caractérisée en ce que** ledit composé de faible solubilité dans l'eau représente de 0.001 à 30% en poids, et préférentiellement de 0,05 à 15% en poids par rapport au poids total de la composition.

4. Composition selon l'une quelconque des revendications 1 à 3 , **caractérisée en ce que** ledit dérivé lipophile d'acide aminé représente de 0,01 à 90% en poids, de préférence de 0,1 à 50% en poids et de manière encore plus préférée de 0,1 à 30% en poids par rapport au poids total de la composition.

5. Composition selon l'une des revendications, dans laquelle le dérivé d'acide salicylique est choisi parmi les dérivés n-octanoyl-5-salicylique, n-décanoyl-5-salicylique, n-dodécanoyl-5-salicylique, n-octyl-5-salicylique, n-heptyloxy-5-salicylique, n-heptyloxy-4-salicylique, 5-tert-octylsalicylique, 3-tert-butyl-3-méthylsalicylique, 3-tert-butyl-6-méthylsalicylique, 3,5-diisopropylsalicylique, 5-butoxysalicylique, 5-octyloxysalicylique, propanoyl-5-salicylique, n-hexadecanoyl-5-salicylique, n-olécyl-5-salicylique, benzoyl-5-salicylique, leurs sels monovalents et divalents et leurs mélanges.

6. Composition selon la revendication précédente dans laquelle le dérivé d'acide salicylique est l'acide n-octanoyl-5-salicylique.

7. Composition selon l'une des revendications précédentes dans laquelle le dérivé d'acide salicylique représente de 0,001 à 15%, de préférence de 0.1 à 5% en poids par rapport au poids total de la composition.

8. Utilisation cosmétique de la composition selon l'une quelconque des revendications précédentes, pour la protection, le soin, le nettoyage et/ou le maquillage de la peau et/ou des muqueuses et/ou des fibres kératiniques.

9. Utilisation de la composition selon l'une quelconque des revendications 1 à 7 pour la fabrication d'une composition thérapeutique dermatologique destinée à lutter contre les signes du vieillissement cutané et/ou lutter contre l'acné et/ou lutter contre les désordres cutanés.

10. Procédé de traitement cosmétique pour lutter contre les signes du vieillissement cutané et/ou améliorer l'éclat du teint, et/ou lisser la peau du visage et/ou du corps, et/ou traiter les rides et les ridules de la peau, et/ou stimuler le processus de renouvellement épidermique, et/ou dépigmenter et/ou blanchir la peau consistant à appliquer sur la peau une composition cosmétique selon l'une quelconque des revendications 1 à 7.

## Claims

1. Cosmetic or dermatological composition comprising, in a physiologically acceptable medium, at least one compound of low solubility in water, i.e. having a solubility in water at 25°C of less that 1% by weight and such that $\delta a > 6\ J^{1/2}\ cm^{-3/2}$, the said compound of low solubility in water being chosen from salicylic acid derivatives of formula I' or a monovalent or divalent salt or mixtures of derivatives of formula I':

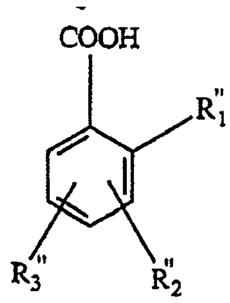

(I')

in which

$R''_1$ represents a hydroxyl radical or an ester of formula

$$-O-CO-R''_4$$

in which $R''_4$ is a saturated or unsaturated aliphatic radical containing from 1 to 26 carbon atoms and preferably from 1 to 18 carbon atoms, or an amine or thiol function optionally substituted with an alkyl radical containing from 1 to 18 carbon atoms and preferably from 1 to 12 carbon atoms,
$R''_2$ and $R''_3$, independently of each other, are in one of the positions 3, 4, 5 or 6 on the benzene nucleus and represent, independently of each other, a hydrogen atom or a radical:

$$-(O)n-(CO)_m-R''_5$$

in which n and m, independently of each other, are each an integer equal to 0 or 1, on condition that $R''_2$ and $R''_3$ are not simultaneously hydrogen atoms,
and $R''_5$ represents a hydrogen, a saturated, linear, branched or cyclized aliphatic radical containing from 1 to 18 carbon atoms, an unsaturated radical containing from 3 to 18 carbon atoms, bearing one to nine conjugated or non-conjugated double bonds, the radicals possibly being substituted with at least one substituent chosen from halogen atoms (fluorine, chlorine, bromine or iodine), trifluoromethyl radicals, hydroxyl radicals in free form or esterified with an acid containing from 1 to 6 carbon atoms, or carboxyl radicals in free form or esterified with a lower alcohol containing from 1 to 6 carbon atoms, or an aromatic radical containing from 6 to 10 carbon atoms, and at least one lipophilic amino acid derivative which is an amino acid ester of formula:

$$R'_1(CO)N(R'_2)CH(R'_3)(CH_2)n(CO)OR'_4$$

in which:

n is an integer equal to 0, 1 or 2,
$R'_1$ represents a linear or branched $C_5$ to $C_{21}$ alkyl or alkenyl radical,
$R'_2$ represents a hydrogen atom or a $C_1$ to $C_3$ alkyl group,
$R'_3$ represents a radical chosen from the group formed by a hydrogen atom, a methyl group, an ethyl group and a linear or branched $C_3$ or $C_4$ alkyl radical,
$R'_4$ represents a linear or branched $C_1$ to $C_{10}$ alkyl or $C_2$ to $C_{10}$ alkenyl radical or a sterol residue.

**2.** Composition according to Claim 1, such that the said amino acid ester is isopropyl N-lauroyl sarcosinate

$$CH_3-(CH_2)_{10}CO-N(CH_3)-CH_2-COO-CH-(CH_3)_2.$$

**3.** Composition according to either of Claims 1 and 2, **characterized in that** the said compound of low solubility in water represents from 0.001% to 30% by weight and preferably from 0.05% to 15% by weight relative to the total

weight of the composition.

4. Composition according to any one of Claims 1 to 3, **characterized in that** the said lipophilic amino acid derivative represents from 0.01% to 90% by weight, preferably from 0.1% to 50% by weight and even more preferably from 0.1% to 30% by weight relative to the total weight of the composition.

5. Composition according to one of Claims 1 to 4, in which the salicylic acid derivative is chosen from 5-n-octanoylsalicylic, 5-n-decanoylsalicylic, 5-n-dodecanoylsalicylic, 5-n-octylsalicylic, 5-n-heptyloxysalicylic, 4-n-heptyloxysalicylic, 5-tert-octylsalicylic, 3-tert-butyl-5-methylsalicylic, 3-tert-butyl-6-methylsalicylic, 3,5-diisopropylsalicylic, 5-butoxysalicylic, 5-octyloxysalicylic, 5-propanoylsalicylic, 5-n-hexadecanoylsalicylic, 5-n-oleoylsalicylic and 5-benzoylsalicylic derivatives, monovalent and divalent salts thereof, and mixtures thereof.

6. Composition according to the preceding claim, in which the salicylic acid derivative is 5-n-octanoylsalicylic acid.

7. Composition according to one of the preceding claims, in which the salicylic acid derivative represents from 0.001% to 15% and preferably from 0.1% to 5% by weight relative to the total weight of the composition.

8. Cosmetic use of the composition according to any one of the preceding claims, for protecting, caring for, cleansing and/or making up the skin and/or mucous membranes and/or keratin fibres.

9. Use of the composition according to any one of Claims 1 to 7, for the manufacture of a therapeutic dermatological composition for combating the signs of ageing of the skin and/or for combating acne and/or for combating skin disorders.

10. Cosmetic treatment process for combating the signs of ageing of the skin and/or for improving the radiance of the complexion and/or for making facial and/or body skin smooth and/or for treating wrinkles and fine lines of the skin and/or for stimulating the process of epidermal renewal and/or for depigmenting and/or bleaching the skin, which consists in applying to the skin a cosmetic composition according to any one of Claims 1 to 7.

## Patentansprüche

1. Kosmetische oder dermatologische Zusammensetzung, die in einem physiologisch akzeptablen Medium mindestens eine Verbindung mit geringer Löslichkeit in Wasser, d.h. einer Löslichkeit in Wasser bei 25 °C unter 1 Gew.-% und so, dass $\delta a > 6\ J^{1/2}\ cm^{-3/2}$, wobei die Verbindung mit einer geringen Löslichkeit in Wasser unter den Salicylsäurederivaten der Formel (I') oder ihren einwertigen Salzen, zweiwertigen Salzen oder Gemischen von Derivaten der Formel (I') ausgewählt ist:

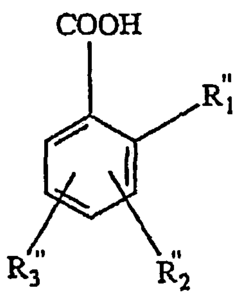

(I')

in der Formel:

$R''_1$ bedeutet eine Hydroxygruppe oder einen Ester der Formel O-CO-$R''_4$, wobei $R''_4$ eine gesättigte oder ungesättigte, aliphatische Gruppe mit 1 bis 26 Kohlenstoffatomen und vorzugsweise 1 bis 18 Kohlenstoffatomen,

eine Amino- oder Thiofunktion bedeutet, die gegebenenfalls mit einer Alkylgruppe substituiert ist, die 1 bis 18 und vorzugsweise 1 bis 12 Kohlenstoffatome enthält;
R"$_2$ und R"$_3$ befinden sich unabhängig voneinander an dem Benzolring in einer der Positionen 3, 4, 5 oder 6 und bedeuten unabhängig voneinander ein Wasserstoffatom oder eine Gruppe:

$$-(O)_n-(CO)_m-R''_5,$$

wobei n und m jeweils unabhängig voneinander eine ganze Zahl 0 oder 1 bedeuten, mit der Maßgabe, dass R"$_2$ und R"$_3$ nicht gleichzeitig Wasserstoffatome bedeuten,
und R"$_5$ bedeutet: Wasserstoff, eine gesättigte lineare, verzweigte oder cyclische, aliphatische Gruppe mit 1 bis 18 Kohlenstoffatomen, eine ungesättigte Gruppe, die 3 bis 18 Kohlenstoffatome aufweist und 1 bis 9 Doppelbindungen besitzt, die konjugiert oder nicht konjugiert sind, wobei die Gruppen mit mindestens einem Substituenten substituiert sein können, der unter den Halogenatomen (Fluor, Chlor, Brom, Iod), Trifluormethylgruppen, einer Hydroxygruppe, die in freier Form vorliegt oder mit einer Säure mit 1 bis 6 Kohlenstoffatomen verestert ist, oder einer Carboxygruppe, die in freier Form vorliegt oder mit einem Alkohol mit 1 bis 6 Kohlenstoffatomen verestert ist, ausgewählt ist, oder eine aromatische Gruppe mit 6 bis 10 Kohlenstoffatomen,

und mindestens ein lipophiles Aminosäurederivat enthält, bei dem es sich um einen Aminosäureester der folgenden Formel handelt:

$$R'_1(CO)N(R'_2)CH(R'_3)(CH_2)_n(CO)OR'_4,$$

worin bedeuten:

n 0 oder eine ganze Zahl 1 oder 2,
R'$_1$ eine geradkettige oder verzweigte Alkyl- oder Alkenylgruppe mit 5 bis 21 Kohlenstoffatomen,
R'$_2$ ein Wasserstoffatom oder eine $C_{1-3}$-Alkylgruppe,
R'$_3$ eine Gruppe, die unter einem Wasserstoffatom, Methyl, Ethyl, einer geradkettigen oder verzweigten Alkylkette mit 3 oder 4 Kohlenstoffatomen ausgewählt ist,
R'$_4$ eine geradkettige oder verzweigte $C_{1-10}$-Alkylgruppe oder eine geradkettige oder verzweigte $C_{2-10}$-Alkenylgruppe oder einen Sterolrest.

2. Zusammensetzung nach Anspruch 1, wobei es sich bei dem Aminosäureester um das Isopropyl-N-lauroylsarcosinat handelt:

$$CH_3-(CH_2)_{10}CO-N(CH_3)-CH_2-COO-CH-(CH_3)_2.$$

3. Zusammensetzung nach einem der Ansprüche 1 oder 2, **dadurch gekennzeichnet, dass** die Verbindung mit geringer Löslichkeit in Wasser 0,001 bis 30 Gew.-% und vorzugsweise 0,05 bis 15 Gew.-%, bezogen auf das Gesamtgewicht der Zusammensetzung, ausmacht.

4. Zusammensetzung nach einem der Ansprüche 1 bis 3, **dadurch gekennzeichnet, dass** das lipophile Aminosäurederivat 0,01 bis 90 Gew.-%, vorzugsweise 0,1 bis 50 Gew.-% und noch bevorzugter 0,1 bis 30 Gew.-%, bezogen auf das Gesamtgewicht der Zusammensetzung, ausmacht.

5. Zusammensetzung nach einem der vorhergehenden Ansprüche, wobei das Salicylsäurederivat unter 5-*n*-Octanoylsalicylsäurederivaten, 5-*n*-Decanoylsalicylsäurederivaten, 5-*n*-Dodecanoylsalicylsäurederivaten, 5-*n*-Octylsalicylsäurederivaten, 5-*n*-Heptyloxysalicylsäurederivaten, 4-*n*-Heptyloxysalicylsäurederivaten, 5-*tert*-Octylsalicylsäurederivaten, 3-*tert*-Butyl-5-methylsalicylsäurederivaten, 3-*tert*-Butyl-6-methylsalicylsäurederivaten, 3,5-Diisopropylsalicylsäurederivaten, 5-Butoxysalicylsäurederivaten, 5-Octyloxysalicylsäurederivaten, 5-Propanoylsalicylsäurederivaten, 5-*n*-Hexadecanoylsalicylsäurederivaten, 5-*n*-Oleoylsalicylsäurederivaten und 5-Benzoylsalicylsäurederivaten, einwertigen und zweiwertigen Salzen dieser Verbindungen und deren Gemischen ausgewählt ist.

6. Zusammensetzung nach dem vorhergehenden Anspruch, da wobei das Salicylsäurederivat die 5-n-Octanoylsalicylsäure ist.

7. Zusammensetzung nach einem der vorhergehenden Ansprüche, wobei das Salicylsäurederivat 0,001 bis 15 Gew.-%, vorzugsweise 0,1 bis 5 Gew.-%, bezogen auf das Gesamtgewicht der Zusammensetzung, ausmacht.

**8.** Kosmetische Verwendung der Zusammensetzung nach einem der vorhergehenden Ansprüche für den Schutz, die Pflege, die Reinigung und/oder zum Schminken der Haut und/oder der Schleimhäute und/oder der Keratinfasern.

**9.** Kosmetische Verwendung der Zusammensetzung nach einem der Ansprüche 1 bis 7 für die Herstellung einer therapeutischen dermatologischen Zusammensetzung, die dazu vorgesehen ist, die Anzeichen der Hautalterung zu bekämpfen und/oder Akne zu bekämpfen und/oder Hautstörungen zu bekämpfen.

**10.** Verfahren zur kosmetischen Behandlung, um die Anzeichen der Hautalterung zu bekämpfen und/oder für einen strahlenderen Teint zu sorgen und/oder die Haut des Gesichts und/oder des Körpers zu glätten und/oder die Falten und Fältchen der Haut zu behandeln und/oder den Prozess der Erneuerung der Epidermis zu stimulieren und/oder die Haut zu depigmentieren und/oder zu bleichen, das darin besteht, auf die Haut eine kosmetische Zusammensetzung nach einem der Ansprüche 1 bis 7 aufzutragen.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- FR 2796550
- EP 1044676 A
- EP 0928608 A
- EP 662318 A
- EP 0662318 A
- EP 987011 A
- JP 2295912 A


### Littérature non-brevet citée dans la description


- **D.W.VAN KREVELEN.** Properties of Polymers. Elsevier, 1990, 200
- **FEDORS.** *Polymer Engineering and Science,* Février 1974, vol. 14 (2
- **D.W.VAN KREVELEN.** Properties of Polymers. Elsevier, 1990
- **R.F. FEDORS.** A method for estimating both the solubility parameters and molar volumes of liquids. *Polymer Engineering and Science,* Février 1974, vol. 14 (2), 147-154